# EUROPEAN PATENT APPLICATION

(11) **EP 1 013 281 A1**
(43) Date of publication of application: **28.06.2000**
(21) Application number: 98123817.3
(22) Date of filing: 15.12.1998
(51) Int. Cl.: A61K 38/24

(54) **hCG therapy for the treatment of breast cancer**

(71) Applicant: Applied Research Systems ARS Holding N.V., Curacao (AN)
(72) Inventor: De Luca, Giampiero, 1208 Geneva (CH)
(74) Representative: Pieraccioli, Daniele

(57) **Abstract**

Breast cancer is treated by human Chorionic Gonadotropin (hCG), preferably in conjunction antiestrogen therapy.More preferably, antiestrogen treatment is sequential to treatment with hCG.

## Description

This invention relates to the field of cancer therapy. More particularly, the invention relates to the treatment of breast cancer by administration of human Chorionic Gonadotropin (hCG), preferably in conjunction with an antiestrogen.

A preferred embodiment comprises the sequential administration of hCG and antiestrogen.

As a further embodiment, the present invention comprises the combined use of hCG and Type I Interferon in conjunction with an antiestrogen.

Breast cancer caused the death of a quarter of a million women worldwide for many years and has been estimated to be the leading cause of death in women aged between 35 to 54, being second only to cardiovascular diseases in women aged over 55 (LOGAN W.P.D.: Cancer of the female breast. International mortality trends. W.H.O. Stat. Rep. 28:232, 1975).

Breast cancer accounts for 27% of all malignancies around the world. Historically, the first to discover the role played by endocrine treatment in breast cancer was BEATSON (1896) who observed that breast cancer in pre-menopausal women undergoes remission after oophorectomy.

This finding, subsequently confirmed by other scientists, supported the evidence that at least some breast tumors are directly dependent on hormones for their growth and created interest in the therapeutic approach of endocrine organ ablation for the purpose of removing the endogenous source of hormones.

As drugs specifically antagonizing the estrogen action were discovered, they became an attractive alternative to surgical ablation.

Several anti-estrogen compounds have been tested in pre- and post-menopausal women in phase I and II clinical trials. So far, Tamoxifen has proved to be the drug best approaching the effectiveness of surgical endocrine therapy and the one that is substantially free from serious side effects.

A comprehensive review of the therapeutic efficacy of antiestrogens in the treatment of breast cancer is LEGHA S.S. and CARTER S.K.: Antiestrogens in the treatment of breast cancer (Cancer Treat. Rev. 3:205, 1976.)

Another review more specifically related to clinical experience with Tamoxifen is that of PATTERSON J.S., et al: A review of the International clinical experience with Tamoxifen. (Jpn. J. Cancer clin. 11 (Suppl.): 157, 1981).

Approximately one-third of women with breast cancer responds to antiestrogen-based hormonal therapy, while an increase up to 70% of response is expected in patients with receptor-rich tumours. In fact, estrogen receptor (ER) status has been demonstrated to be predictive of response in breast cancer patients (ALLEGRA J.C.: Reviews on Endocrine related cancer. Paterson AHG, Lees AW eds Suppl. 14:115, 1984).

Chorionic gonadotropin is a glycoprotein hormone composed of two non-covalently linked (α and β) subunits. (Labrie : Glycoprotein hormones: gonadotropins and thyrotropin. In: Hormones - From Molecules to Disease. Beanlien EE and Kelly PA - Chapman and Hall, New York and London, pp 257-275, 1990).

It is synthesized early in pregnancy by the developing embryo and throughout the gestational process by the syncytiotrophoblast of the placenta, and it is secreted in urine.

Human chorionic gonadotropin is obtained from the urine of pregnant women for both experimental and clinical uses. The hormone can also be prepared via the recombinant route (WO 85/01959) The main known function of hCG is the stimulation of gonadal steroid hormone production through its interaction with the LH/CG receptor, which is present in the granulosa cells of the ovary in the female and in the testicular Leydig cells in the male.

Recent studies demonstrated that hCG is a potent chemopreventive agent that inhibits mammary tumorigenesis through the induction of differentiation. (Russo IH, et al. J. Natl. Cancer Inst. 82:1286-1289, 1990). It also inhibits the proliferation of normal and neoplastic human breast epitelial cells (Alvarado ME, et at, In Vitro 30A: 4-8, 1994). Recent studies indicate that urinary hCG from various sources has an inhibitory effect on neoplastic cell lines from various organs or systems (Gill PS, et at, J. Natl Cancer Inst. 89: 1797-1802, 1997) (Albini A. Et al, AIDS 11: 713-721, 1997)

We have now found that hCG is effective in the treatment of conclamate human mammary tumors. We have also found that the combined treatment with hCG and anti-estrogen has been shown to be-much more effective in reducing the growth of breast cancer cells. Particularly good results are obtained when the administration of the antiestrogen is sequential to a treatment with hCG.

It is therefore an object of this invention to provide the use of hCG in the manufacture of a medicament for the treatment of breast cancer. It is a further object of this invention to provide the use of hCG in conjunction with an anti estrogen in the manufacture of a medicament for the treatment of breast cancer.

A further object of this invention is to provide a method of treating breast cancer by the simultaneous, sequential or separate administration of hCG and anti-estrogen. Particularly preferred is the sequential administration. A pharmaceutical composition containing hCG and antiestrogen is also within the scope of the present invention.

Within the meaning of hCG the full dimer hCG, its beta subunit and any fragment thereof having anticancer activity are included. HCG can be either "native", that is obtained from natural human sources or cell lines, or "recombinant", that is obtained from genetically engineered or otherwise modified bacterial, yeast or eukaryotic cells. HCG can be administered intramuscularly and, preferably, subcutaneously.

We have also found that the additional use of a Type I Interferon in the treatment of breast cancer with hCG and an antiestrogen further reduces the growth of breast cancer cells.

Interferons (IFNs) are a well-known family of proteins which have been shown to possess both antiviral and cell growth inhibitory effects.

The term "Type I Interferon" comprises IFN alpha from leukocytes or from lymphoblasts, IFN beta from fibroblasts and IFN omega.

Interferons have a wide range of cellular effects on cancer, as well as on normal cells, including such effects on cell phenotype as antigen expression, cell receptors and so on. Experimental evidence exists that type I IFN modifies the hormone receptor level in breast cancer tissue cells. For example, POUILLART T., et al, in "Administration of fibroblast interferon to patients with advanced breast cancer: possible effects on skin metastasis and on hormone receptors" (Eur. J. Caner Clin. Oncol. 18:929-935, 1982) described the effect of human fibroblast interferon administered to patients with metastasized breast cancer and found an increase of the receptors for estrogens and progestogens.

It is thus a further object of the present invention the use of hCG and Type I Interferon in conjunction with an antiestrogen in the manufacture of a medicament of a medicament for the treatment of breast cancer as well as a pharmaceutical composition therefor, for the simultaneous, separate or sequential use of its active components in the treatment of breast cancer.

Although Tamoxifen is the preferred antiestrogen, other substances having analogous activity are within the meaning of "antiestrogen" in accordance with the present invention. Suitable examples are found, e.g., in the above mentioned review by Legha and Carter.

The finding on which this invention is based is that the new combined treatment of breast cancer cells gives results that could not be predicted from the known antiproliferative effect of hCG or the demonstrated efficacy on an antiestrogen when used separately.

Preliminary in vitro experiments were carried out on CG-5 cells, a variant of the MCF-7 cell line characterized by a high degree of estrogen responsiveness and an appreciable content of estrogen, androgen, glucocorticoid and progesterone receptors - Natoli C. et al: Two new estrogen supersensitive variants of the MCF-7 human breast cancer cell line - (Breast Cancer Res. Treat. 3, 23-32, 1983).

Cells were routinely cultured in Dulbecco's modified Eagle's medium (DMEM) supplemented with 10% fetal calf serum (FCS) and antibiotics.

For cell growth experiments cells were plated out at the density of 50,000 cells /ml in the medium described above. 24 hours later, the medium was replaced with fresh medium containing 5% charcoals-treated FCS CH-FCS) plus a fixed concentration (10⁻⁷ M) of Tamoxifen (TAM) and various concentrations (from 10 to 10000 IU/ml) of hCG. Medium was renewed every 3 days.

In the experiments concerning the efface of hCG and TAM sequentially added to CG-5 cells, cells were plated out at 50,000 cells/ml, as described above, and 24 hours later DMEM was changed with fresh medium containing 10 to 10000 IU/ml of hCG. For each concentration of hCG a different number of plates were prepared in order to have, at the end of the treatment, a sufficient number of cells to be replaced (as hCG has an inhibitory effect itself). After 1 week of exposure to hCG, CG-5 cells were plated in medium supplemented which 10% FCS and antibiotics, and 24 hours later DMEM was replaced by fresh medium supplemented with 5% CE-FCS and a fixed concentration of TAM (10⁻⁷ M). Medium was renewed every 3 days.

In all the experiments performed cells were counted, after 3 to 6 days, with the use of an hemocytometer.

The addition to CG-5 cultures of a fixed concentration of TAM (10⁻⁷ M), combined to concentrations ranging from 10 to 1000 IU/ml of hCG, produces an inhibition of cell proliferation which is not related to the dose of hCG but is higher than that induced by TAM alone, even at the lowest concentration of hCG (about 50% with respect to control) on the third day from the addition of the two drugs to the culture medium.

When cells are treated for 6 days with the combination TAM- hCG, the inhibition of cell proliferation becomes dependent on the dose of hCG and reaches 65% with respect to controls at 1000 IU/ml of the drug.

If CG-5 cells are pretreated with different concentrations of hCG and subsequently exposed to 10⁻⁷ M TAM, 3 days after the addition of the antiestrogen to the culture medium, a relevant inhibition of cell proliferation is seen (approximately 50% with respect to control) in the cells which received the highest concentration of hCG.

On the sixth day after the addition of TAM to the culture medium, the most pronounced inhibition of cell proliferation is obtained in CG-S cells pretreated with the lowest concentration of hCG (about 65% with respect to control) and it remains unmodified in cells pretreated with increasing doses of the drug.

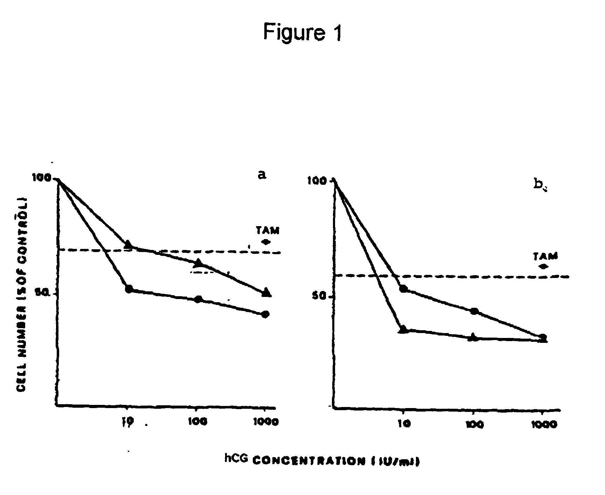

Results are summarised in figure 1, a and b, which illustrate graphically the comparison between the different modalities (combined or sequential treatment) used to study the effect of Tamoxifen and hCG on cell growth.

Graphs a and b demonstrate the effect of hCG and Tamoxifen added simultaneously (large dot symbol) or sequentially (triangular symbol) to CG-5 cells on the third (a) and sixth (b) day from the addition of the compounds to the culture medium. In the case of sequential administration cells were pretreated with the hCG concentration indicated in the figure and then exposed to Tamoxifen. The dotted line ( -- ) represents the effect of 10⁻⁷ M Tamoxifen alone, evaluated in parallel experiments not reported in the text.

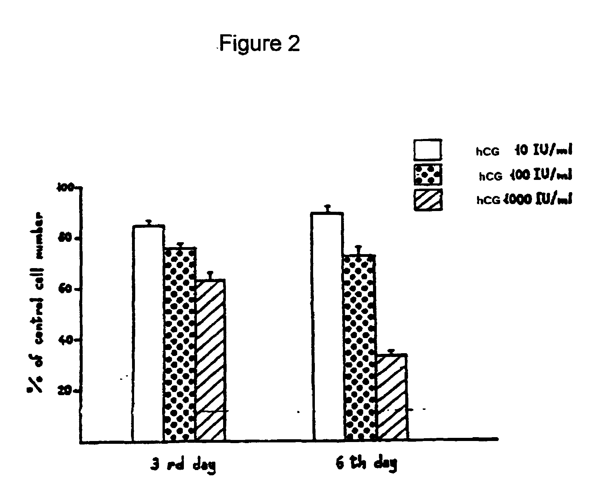

Figure 2 shows the effect of hCG alone on the growth of CG-5 cells cultured in identical experimental conditions. In this case the inhibition of cell proliferation is evident after three days of exposure to the hCG starting from the concentration of 100 IU/ml. After six days of treatment with hCG, the inhibitory effect on cell proliferation significantly increases only at the maximum dose of 1000 IU/ml.

The comparison between figures 1 and 2 clearly shows that such low doses of hCG as 10 IU/ml result efficacious when combined with the anti-estrogen, whereas the same doses are practically ineffective if hCG is used alone.

Similar conclusions are reached if the effect of Tamoxifen alone is compared with the combined effect of Tamoxifen with hCG.

The efficacy of the combined therapy according to this invention is established by clinical trials conducted in patients responding to the following criteria:
- Patients with superficial biopsiable, histologically confirmed advanced breast cancer:
- Patients in postmenopausa:
- ER/PR status: positive or unknown (provided at least 2 years of disease free interval);
- No concurrent radiotherapy or chemotherapy;
- No brain metastases.

Each patient receives intramuscular injections of hCG doses ranging from 2 to 10 million International Units and is given orally a fixed dose of the antiestrogen corresponding to the dose known to be optimal for breast cancer treatment. For example, in the case of Tamoxifen, the most suitable daily dose is 30 milligrams.

Examples of suitable treatment schedules are as follows:
1)
   - hCG administered three times a week for two to four weeks;
   - Antiestrogen administered daily for 8 weeks starting immediately after discontinuation of hCG administration.
2)
   - hCG administered three times a week for two to four weeks;
   - Antiestrogen administered daily for 12 weeks starting on the same day the hCG treatment starts.
3)
   - hCG administered daily for one to four weeks;
   - Antiestrogen administered daily for 12 weeks starting the day after the last hCG administration.
   4)
   - The same treatment scheduled (3) above with once a week addition- al administration of hCG in the period of antiestrogen therapy.

Additional i.m. administration of Interferon beta (Rebif, Ares-Serono) at a dose ranging from 10 to 10 million IU resulted in optimal response in the above experiments for breast cancer treatment.

The main criterion of evaluation is the evidence of response documented on the basis of tumor regression assessed by measurement of palpable lesions and changes in X-Ray or computerized tomograghy scan or ultrasonic echography appearances.

The increase of Estrogen receptors in the patients is also taken into consideration as a laboratory datum supplementing clinically assessed responsiveness.

The efficacy of the combined hCG / antiestrogen treatment in breast cancer is thus established. Optimal dose for hCG resulted to be in the range 100-1000 IU/day

Various changes and modifications can be made in the therapeutic method of the present invention without departing from the spirit and scope thereof. The embodiments described herein are for the purpose of illustrating the invention but are not intended to limit it.

## Claims

1. Use of hCG in the manufacture of a medicament for the treatment of breast cancer.

2. Use of hCG in conjunction with an antiestrogen in the manufacture of a medicament for the treatment of breast cancer.

3. Use according to claim 2 in which hCG is used simultaneously, sequentially or separately with the antiestrogen.

4. Use according to claims 2 or 3, wherein the antiestrogen is Tamoxifen.

5. Use according to claim 4, wherein Tamoxifen is administered orally in a daily amount of about 30 milligrams.

6. Use according to claims 1 to 5, wherein hCG is administered in an amount of 2 to 10 million International Units.

7. Use according to claim 6, wherein hCG is administered in an amount of 100 to 1000 International Units.

8. Use according to claims 1 to 7, wherein hCG is administered subcutaneously.

9. Use according to any of claims 2 to 8, which additionally comprises the use of Type I Interferon in conjunction with hCG and an antiestrogen.

10. Pharmaceutical composition containing hCG and an antiestrogen, in the presence of one or more pharmaceutically acceptable excipients, for the simultaneous, sequential or separate use of its active components in the treatment of breast cancer.

11. Pharmaceutical composition according to claim 10 wherein hCG is administered in an amount of 2 to 10 million International Units.

12. Pharmaceutical composition according to claim 11 wherein hCG is administered subcutaneously.

13. Pharmaceutical composition according to any of claims 10 to 12, wherein the antiestrogen Tamoxifen.

14. Pharmaceutical composition according to claim 13, wherein Tamoxifen is administered orally in a daily amount of about 30 milligrams.

15. Pharmaceutical composition according to any of claims 10 to 14, which additionally comprises the use of Type I Interferon in conjunction with hCG and an antiestrogen.

16. A method of inhibiting the proliferation of breast cancer cells which comprises administering a host in need thereof an effective inhibiting amount of hCG.

17. A method of inhibiting the proliferation of breast cancer cells which comprises administering a host in need thereof an effective inhibiting amount of a combination of hCG and antiestrogen.

18. The method according to claim 17, additionally comprising administration of a Type I Interferon to a host in need thereof.
